# EUROPEAN PATENT APPLICATION

(11) **EP 4 566 625 A1**
(43) Date of publication of application: **11.06.2025**
(21) Application number: 23850024.3
(22) Date of filing: 28.07.2023
(51) Int. Cl.: A61K 39/215, A61K 35/76, A61K 39/00, A61K 39/39, A61P 31/14, A61P 37/04, C12N 7/02

(54) **METHOD FOR MANUFACTURING INACTIVATED SARS-COV-2 VACCINE, INACTIVATED SARS-COV-2 VACCINE, METHOD FOR PURIFYING SARS-COV-2 OR INACTIVATED SARS-COV-2, AND SARS-COV-2 ANTIGEN COMPOSITION OR INACTIVATED SARS-COV-2 ANTIGEN COMPOSITION**

(30) Priority: 03.08.2022 JP 2022124134
(71) Applicant: KM Biologics Co., Ltd., Kumamoto-shi, Kumamoto 860-8568 (JP)
(72) Inventor: OKUMURA Minako, Kikuchi-shi, Kumamoto 869-1298 (JP); KOUJIMOTO Yamato, Kikuchi-shi, Kumamoto 869-1298 (JP); DAIMON Kazuhiro, Kikuchi-shi, Kumamoto 869-1298 (JP); SAKAMOTO Takuya, Kikuchi-shi, Kumamoto 869-1298 (JP); MIENO Ryoko, Kikuchi-shi, Kumamoto 869-1298 (JP); ODA Ryusei, Kikuchi-shi, Kumamoto 869-1298 (JP); NAKAJIMA Shingo, Kikuchi-shi, Kumamoto 869-1298 (JP); ENDO Masafumi, Kikuchi-shi, Kumamoto 869-1298 (JP)
(74) Representative: Boult Wade Tennant LLP
(86) International application number: PCT/JP2023/027808
(87) International publication number: WO 2024/029469

(57) **Abstract**

The present invention relates to a production method of an inactivated SARS-CoV-2 vaccine, the method including: a step of bringing a SARS-CoV-2 containing solution or an inactivated SARS-CoV-2 containing solution into contact with a cellulose sulfate ester gel at a pH of 8 or more and 10 or less to adsorb the SARS-CoV-2 or the inactivated SARS-CoV-2 to the gel; then removing impurities; and then eluting and recovering the SARS-CoV-2 or the inactivated SARS-CoV-2.

## Description

### Technical Field

The present invention relates to a production method of an inactivated SARS-CoV-2 vaccine, an inactivated SARS-CoV-2 vaccine, a purification method of SARS-CoV-2 or an inactivated SARS-CoV-2, and a SARS-CoV-2 antigen composition or an inactivated SARS-CoV-2 antigen composition.

### Background Art

The novel coronavirus infection that occurred in 2019 is called COVID-19, the international official name given by the World Health Organization (WHO), and caused by a virus called SARS-CoV-2. COVID-19 is a respiratory tract infectious disease typically exhibiting symptoms such as headache, loss of olfactory and/or taste, nasal congestion and rhinorrhea, cough, muscle pain, sore throat, fever, diarrhea, and dyspnea. People infected with COVID-19 are often with mild symptoms involving no symptoms or cold-like symptoms, and heal spontaneously. However, acute respiratory distress syndrome, sepsis, and/or multiple organ failure are involved in severe cases. SARS-CoV-2 is a positive-sense single-stranded RNA virus that belongs to the Orthocoronavirus subfamily and has a diameter of 50 to 200 nm.

Vaccination is one of the preventive methods against COVID-19. As vaccines against SARS-CoV-2, various vaccines such as mRNA vaccines, viral vector vaccines, and recombinant protein vaccines have been developed and approved, and some of them have already been put on the market (Non Patent Literature 1). Also in Japan, mRNA vaccines and viral vector vaccines have been inoculated to many people of 5 years of age or older, and it is expected to reduce the number of severe people and the number of dead people by preventing infection, severity, and onset. On the other hand, as for these vaccines, side reactions after inoculation have been reported, and the side reactions includes pain at the inoculation site, headache, malaise, and muscle pain, and in very rare cases, includes anaphylaxis after inoculation, and thrombosis, pericarditis, and myocarditis, depending on the vaccine type.

On the other hand, an inactivated vaccine is also known as a vaccine other than the types described above. The inactivated vaccine is used as, for example, a seasonal influenza vaccine, a Japanese encephalitis vaccine, a four-type combination vaccine, and the like. The inactivated vaccine is a vaccine produced using a pathogen (virus or the like) that has lost infectivity or a component of the pathogen (part of virus or the like), and is generally known to have less side reactions and high safety. Therefore, it is expected that an inactivated SARS-CoV-2 vaccine can also be inoculated into children under 5 years of age.

The method for producing an inactivated vaccine includes, as an embodiment, a method in which a target virus is proliferated using established cells as a virus culture substrate (host cell), and then the proliferated virus is inactivated and purified to obtain a purified inactivated virus as a virus antigen composition for vaccine production. This production method requires establishment of a proliferation method of a virus for achieving high productivity and establishment of a purification method of an inactivated virus for achieving further safety. For an inactivated SARS-CoV-2 vaccine, purification by ion exchange chromatography and/or size exclusion chromatography is usually employed as a purification method of an inactivated virus (Patent Literatures 1 and 2). There is also a report that purification by affinity chromatography was performed (Patent Literature 3, and Non Patent Literature 2).

### Citation List

### Patent Literature

Patent Literature 1: WO 2021/204825 A
Patent Literature 2: WO 2021/209060 A
Patent Literature 3: WO 2022/38642 A

### Non Patent Literature

Non Patent Literature 1: Clinical Immunology 222(2021)108634 Non Patent Literature 2: Purification Example of Human Coronavirus OC43 with Cellufine Sulfate, Technical Data Sheet from JNC CORPORATION https://wwwjnc-corp.co.jp/fine/jp/cellufine/guide/pdf/affinity/TD_Sulfate_N5_V1_J.pdf (confirmed on June 1, 2022)

### Summary of Invention

### Technical Problem

An object of the present invention is to provide a production method of a high-purity inactivated SARS-CoV-2 vaccine containing a small amount of (especially, containing substantially no) impurities (for example, host cell-derived protein) derived from host cells (for example, established animal cells). In addition, an object of the present invention is to provide a purification method of SARS-CoV-2 or an inactivated SARS-CoV-2 capable of obtaining a high-purity SARS-CoV-2 antigen composition or a high-purity inactivated SARS-CoV-2 antigen composition containing a small amount of (especially, containing substantially no) impurities (for example, host cell-derived protein) derived from host cells (for example, established animal cells). Further, an object of the present invention is to provide an inactivated SARS-CoV-2 vaccine, a SARS-CoV-2 antigen composition, or an inactivated SARS-CoV-2 antigen composition each obtained by the production method or the purification method.

### Solution to Problem

As a result of intensive studies to achieve the above object, the present inventors have found that when SARS-CoV-2 is proliferated using established animal cells (Vero cells) as a virus culture substrate (host cells), and then the inactivated SARS-CoV-2 is purified by cellulose sulfate ester gel adsorption chromatography under conditions of a pH of 8 or more and 10 or less, a more highly purified inactivated SARS-CoV-2 containing substantially no impurities (protein) derived from the virus culture substrate (host cells) can be obtained, and thus have completed the present invention.

The present invention relates to a production method of an inactivated SARS-CoV-2 vaccine, the method comprising: a step of bringing a SARS-CoV-2 comprising solution or an inactivated SARS-CoV-2 comprising solution into contact with a cellulose sulfate ester gel at a pH of 8 or more and 10 or less to adsorb the SARS-CoV-2 or the inactivated SARS-CoV-2 to the gel; then removing impurities; and then eluting and recovering the SARS-CoV-2 or the inactivated SARS-CoV-2.

The production method of the present invention, comprising purifying SARS-CoV-2 or an inactivated SARS-CoV-2 under specific pH conditions by using a cellulose sulfate ester gel, is capable of obtaining an inactivated SARS-CoV-2 vaccine comprising substantially no impurities (for example, host cell-derived protein) derived from host cells (for example, established animal cells).

The present invention also relates to an inactivated SARS-CoV-2 vaccine obtained by the above-described production method, and comprising substantially no host cell-derived protein. The vaccine, obtained by the production method according to the present invention described above, comprises substantially no impurities (for example, host cell-derived protein) derived from host cells (for example, established animal cells), and is further enhanced in safety.

The vaccine may further comprise an adjuvant.

The present invention can be understood also as a purification method of SARS-CoV-2 or an inactivated SARS-CoV-2, the method comprising: a step of bringing a SARS-CoV-2 comprising solution or an inactivated SARS-CoV-2 comprising solution into contact with a cellulose sulfate ester gel at a pH of 8 or more and 10 or less to adsorb the SARS-CoV-2 or the inactivated SARS-CoV-2 to the gel; then removing impurities; and then eluting and recovering the SARS-CoV-2 or the inactivated SARS-CoV-2.

The present invention further relates to a SARS-CoV-2 antigen composition or an inactivated SARS-CoV-2 antigen composition obtained by the above-described purification method, and comprising substantially no host cell-derived protein.

As a result of intensive studies to achieve the above object, the present inventors have found that when SARS-CoV-2 is proliferated using established animal cells (Vero cells) as a virus culture substrate (host cells), and then the inactivated SARS-CoV-2 is adsorbed under conditions of a pH of 7 or more and 8 or less, followed by removing impurities under conditions of a pH of 8 or more and 10 or less, eluted and recovered, and purified through cellulose sulfate ester gel adsorption chromatography, a highly purified inactivated SARS-CoV-2 comprising a small amount of impurities (protein) derived from the virus culture substrate (host cells) can be obtained in a high yield, and thus have completed the present invention.

The present invention relates to a production method of an inactivated SARS-CoV-2 vaccine, the method comprising: a step of bringing a SARS-CoV-2 comprising solution or an inactivated SARS-CoV-2 comprising solution into contact with a cellulose sulfate ester gel at a pH of 7 or more and 8 or less to adsorb the SARS-CoV-2 or the inactivated SARS-CoV-2 to the gel; then removing impurities at a pH of 8 or more and 10 or less; and then eluting and recovering the SARS-CoV-2 or the inactivated SARS-CoV-2.

The production method of the present invention, comprising purifying SARS-CoV-2 or an inactivated SARS-CoV-2 under specific pH conditions by using a cellulose sulfate ester gel, is capable of efficiently producing a high-purity inactivated SARS-CoV-2 vaccine comprising a small amount of impurities (for example, host cell-derived protein) derived from host cells (for example, established animal cells).

The present invention also relates to an inactivated SARS-CoV-2 vaccine obtained by the above-described production method. The vaccine, obtained by the production method according to the present invention described above, comprises a small amount of impurities (for example, host cell-derived protein) derived from host cells (for example, established animal cells), and is enhanced in safety.

In the vaccine, a content of host cell-derived protein may be 30 ng or less with respect to 1 µg of protein comprised in the inactivated SARS-CoV-2 vaccine.

The vaccine may further comprise an adjuvant.

The present invention can be understood also as a purification method of SARS-CoV-2 or an inactivated SARS-CoV-2, the method comprising: a step of bringing a SARS-CoV-2 comprising solution or an inactivated SARS-CoV-2 comprising solution into contact with a cellulose sulfate ester gel at a pH of 7 or more and 8 or less to adsorb the SARS-CoV-2 or the inactivated SARS-CoV-2 to the gel; then removing impurities at a pH of 8 or more and 10 or less; and then eluting and recovering the SARS-CoV-2 or the inactivated SARS-CoV-2.

The present invention further relates to a SARS-CoV-2 antigen composition or an inactivated SARS-CoV-2 antigen composition obtained by the above-described purification method.

In the antigen composition, a content of host cell-derived protein may be 30 ng or less with respect to 1 µg of protein comprised in the SARS-CoV-2 antigen composition or the inactivated SARS-CoV-2 antigen composition.

### Advantageous Effects of Invention

According to the present invention, it is possible to provide a production method of a high-purity inactivated SARS-CoV-2 vaccine containing a small amount of (especially, containing substantially no) impurities (for example, host cell-derived protein) derived from host cells (for example, established animal cells). In addition, according to the present invention, it is possible to provide a purification method of SARS-CoV-2 or an inactivated SARS-CoV-2 capable of obtaining a high-purity SARS-CoV-2 antigen composition or a high-purity inactivated SARS-CoV-2 antigen composition containing a small amount of (especially, containing substantially no) impurities (for example, host cell-derived protein) derived from host cells (for example, established animal cells). Further, according to the present invention, it is possible to provide an inactivated SARS-CoV-2 vaccine, a SARS-CoV-2 antigen composition, or an inactivated SARS-CoV-2 antigen composition each obtained by the production method or the purification method.

The inactivated SARS-CoV-2 vaccine of the present invention is capable of inducing an antibody having neutralizing activity against SARS-CoV-2.

### Brief Description of Drawings

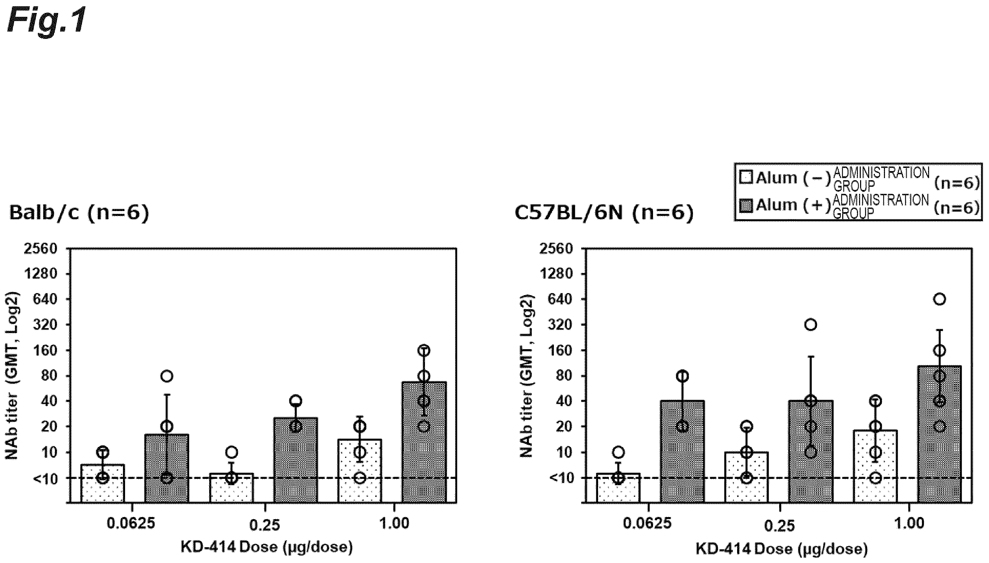

FIG. 1 is a graph showing the results of measured neutralizing antibody value against SARS-CoV-2 in serum in Example 5.

### Description of Embodiments

Hereinafter, embodiments for carrying out the present invention will be described in detail. However, the present invention is not limited to the following embodiments.

### [Production Method of Inactivated SARS-CoV-2 Vaccine]

The production method of an inactivated SARS-CoV-2 vaccine according to the embodiment includes: a step of bringing a SARS-CoV-2 containing solution or an inactivated SARS-CoV-2 containing solution into contact with a cellulose sulfate ester gel at a pH of 8 or more and 10 or less to adsorb the SARS-CoV-2 or the inactivated SARS-CoV-2 to the gel; then removing impurities; and then eluting and recovering the SARS-CoV-2 or the inactivated SARS-CoV-2 (purification step A).

The production method according to the embodiment is characterized in that in the production process of an inactivated SARS-CoV-2 vaccine, the purification step where SARS-CoV-2 or an inactivated SARS-CoV-2 is adsorbed to a cellulose sulfate ester gel and eluted is performed under alkaline conditions (a pH of 8 or more and 10 or less). As a result, the removal efficiency of impurities (for example, host cell-derived protein) in the purification step is improved, and a high-purity inactivated SARS-CoV-2 vaccine substantially containing no impurities can be obtained.

The production method of an inactivated SARS-CoV-2 vaccine according to another embodiment includes: a step of bringing a SARS-CoV-2 containing solution or an inactivated SARS-CoV-2 containing solution into contact with a cellulose sulfate ester gel at a pH of 7 or more and 8 or less to adsorb the SARS-CoV-2 or the inactivated SARS-CoV-2 to the gel; then removing impurities at a pH of 8 or more and 10 or less; and then eluting and recovering the SARS-CoV-2 or the inactivated SARS-CoV-2 (purification step B).

The production method according to another embodiment is characterized in that in the production method of an inactivated SARS-CoV-2 vaccine, in the purification step where SARS-CoV-2 or an inactivated SARS-CoV-2 is adsorbed to a cellulose sulfate ester gel, impurities are removed, and SARS-CoV-2 or an inactivated SARS-CoV-2 is eluted, the adsorption is performed under the condition of around neutral (a pH of 7 or more and 8 or less), and the removal of impurities and the elution are subsequently performed under alkaline conditions (a pH of 8 or more and 10 or less). As a result, the removal efficiency of impurities in the purification step is improved, the yield of SARS-CoV-2 or an inactivated SARS-CoV-2 is further improved, and a high-purity inactivated SARS-CoV-2 vaccine containing a small amount of impurities can be more efficiently produced.

The production method according to the embodiment may further include, in addition to the purification step A or B (hereinafter, may be collectively referred to as "purification step"), at least one step selected from the group consisting of a step of proliferating SARS-CoV-2 (proliferation step), a step of inactivating SARS-CoV-2 (inactivation step), and a step of mixing the purified inactivated SARS-CoV-2 with other components to obtain an inactivated SARS-CoV-2 vaccine (formulation step).

### (Proliferation Step)

The proliferation step is a step of proliferating SARS-CoV-2. SARS-CoV-2 can be proliferated by infecting host cells as a virus culture substrate with SARS-CoV-2 and recovering the SARS-CoV-2 proliferated in the infected host cells.

The host cells used for proliferation of SARS-CoV-2 are not particularly limited as long as they are cells being excellent in proliferation of SARS-CoV-2 and producing a SARS-CoV-2 (antigen) having high immunogenicity. For example, an animal other than the human (for example, chicken) or animal-derived established cells (established animal cells) can be used. Specific examples of the established cells include Calu-3 cells and A549 cells derived from human lung epithelial adenocarcinoma cells, and Vero cells, VeroE6 cells, and VeroE6/TMPRSS2 cells derived from African green monkey kidney. Examples of the host cells suitably used in the production method according to the embodiment include Vero cells.

The viral strain of SARS-CoV-2 used in the production method according to the embodiment is not particularly limited as long as it is a viral strain maintaining an infection defense antigen. Examples of the viral strain of SARS-CoV-2 include the early strain (Wuhan strain) and its mutant strains such as the alpha strain, the beta strain, the gamma strain, the delta strain, and the omicron strain.

When established cells are used as host cells, the medium used for expansion of the host cells (cell proliferation) (hereinafter, may be referred to as "proliferation medium") is appropriately selected from mediums generally used for tissue culture, such as M199-earle base, Minimum essential medium (MEM), Dulbecco minimum essential medium (D-MEM), SC-UCM102 (Nissui), VP-SFM (GIBCO BRL), EX-CELL302 (NICHIREI), EX-CELL293-S (NICHIREI), TFBM-01 (NICHIREI), and ASF104, and is used after an amino acid, a salt, an anti-mold/antibacterial agent, animal serum, or the like is added thereto. As the proliferation medium, preferably used is a D-MEM medium and a medium in which an amino acid, a salt, an anti-mold/antibacterial agent, animal serum, or the like is added to a D-MEM medium.

Examples of the medium used for proliferation of SARS-CoV-2 after SARS-CoV-2 is inoculated into host cells (hereinafter, may be referred to as "maintenance medium") include a serum-free or low-protein concentration medium. As the maintenance medium, the above proliferation medium containing no animal serum can be used, but a medium obtained by adding L-glutamic acid to VP-SFM is preferably used. The use of serum-free medium facilitates purification due to less impurities to be removed and can eliminate the possibility that unknown pathogens are contaminated from serum.

Examples of the proliferation method of host cells include a static culture method, a roller bottle culture method, and a suspension culture method. Among these, tank culture using a microcarrier, which is one of the suspension culture methods, enables high-density culture of host cells, and is suitable as a method for acquiring a large amount of SARS-CoV-2. As a microcarrier used for this purpose, many products are commercially available. For example, Cytodex (registered trademark) I (Cytiva (Global Life Sciences Technologies Japan K.K.)) is preferable for the proliferation of Vero cells. Cytodex (registered trademark) is preferably used at a concentration of 1 g/L or more and 5 g/L or less with respect to proliferation medium.

When host cells are infected with SARS-CoV-2, the number of SARS-CoV-2 per host cell, that is, the multiplicity of infection (MOI) may be, for example, in the range of 0.000001 or more and 0.01 or less, or in the range of 0.00001 or more and 0.001 or less.

In the host cells infected with SARS-CoV-2, the culture temperature and the culture period for the proliferation of SARS-CoV-2 are adjusted by a combination of the type of host cell, SARS-CoV-2 inoculum amount, culture scale, culture method, and the like. For example, the following method is adopted when SARS-CoV-2 is proliferated in Vero cells by static culture or roller bottle culture.

First, Vero cells are cultured in a proliferation medium made of a D-MEM medium to which a non-essential amino acid and bovine serum are added, at a culture temperature of 32°C or more and 38°C or less, preferably 37°C, for a culture period of 2 days or more and 7 days or less, preferably 5 days or more and 7 days or less. Next, when the proliferation of Vero cells reaches a stable phase, the medium is removed by suction, and the Vero cells are washed several times with phosphate buffered saline or the like. Then SARS-CoV-2 is inoculated at an MOI of 0.000001 or more and 0.01 or less, preferably at an MOI of 0.0001, VP-SFM is added as a maintenance medium, and then the Vero cells are cultured at a culture temperature of 32°C or more and 38°C or less for 3 days or more and 8 days or less, and preferably at 37°C for 5 days or more and 7 days or less. The culture thus obtained, that is, the host cell lysate or culture supernatant contains a large amount of SARS-CoV-2.

The SARS-CoV-2 containing solution obtained in the proliferation step (for example, the host cell lysate or culture supernatant) is used to perform the inactivation step or the purification step.

### (Inactivation Step)

The inactivation step is a step of inactivating SARS-CoV-2. The inactivation step can be performed, for example, by bringing SARS-CoV-2 into contact with an inactivating agent. In addition, for example, SARS-CoV-2 may be inactivated by adding an inactivating agent to a culture of host cells infected with SARS-CoV-2. Alternatively, SARS-CoV-2 may be inactivated by adding an inactivating agent to the culture from which impurities have been removed and/or the culture after concentration. Specifically, for example, the culture of host cells infected with SARS-CoV-2 is subjected to crude centrifugation or membrane filtration to remove insoluble matter, then the obtained supernatant is further concentrated through an ultrafiltration membrane having an exclusion limit molecular weight of 100,000 or more and 750,000 or less, preferably 300,000 or more and 750,000 or less to obtain a concentrated solution, and the concentrated solution is used for inactivation.

Examples of the inactivating agent include formaldehyde, paraformaldehyde, glutaraldehyde, dicyclohexylcarbodiimide (DCC), diisopropylcarbodiimide (DIC), 1-ethyl-3-(3-dimethylaminopropyl) carbodiimide hydrochloride, and β-propiolactone. The inactivating agent is preferably β-propiolactone.

The addition amount of the inactivating agent for inactivating SARS-CoV-2 can be set according to the type of the solution containing SARS-CoV-2, the type of the inactivating agent, and the like. For example, when the culture of host cells infected with SARS-CoV-2 is subjected to crude centrifugation or membrane filtration to remove insoluble matter, then the obtained supernatant is further concentrated through an ultrafiltration membrane having an exclusion limit molecular weight of 100,000 or more and 750,000 or less, preferably 300,000 or more and 750,000 or less to obtain a concentrated solution, and the concentrated solution is inactivated by adding β-propiolactone, the addition amount of the inactivating agent may be 0.01 w/v% or more and 0.1 w/v% or less, and is preferably 0.05 w/v%.

The temperature and reaction time (time for bringing into contact with the inactivating agent) during the inactivation of SARS-CoV-2 can be set according to the type of the solution containing SARS-CoV-2, the type and concentration of the inactivating agent, and the like. For example, when the culture of host cells infected with SARS-CoV-2 is subjected to crude centrifugation or membrane filtration to remove insoluble matter, then the obtained supernatant is further concentrated through an ultrafiltration membrane having an exclusion limit molecular weight of 100,000 or more and 750,000 or less, preferably 300,000 or more and 750,000 or less to obtain a concentrated solution, and the concentrated solution is inactivated by adding β-propiolactone, the inactivation can be performed by stirring at a reaction temperature of around 20°C for 16 hours or more.

The inactivation step may be performed before the purification step, or may be performed after the purification step. When the inactivation step is performed before the purification step, the purification step may be performed using the inactivated SARS-CoV-2 containing solution obtained in the inactivation step (for example, a reaction liquid that has been reacted with the inactivating agent).

### (Purification Step)

The purification step A is a step of bringing a SARS-CoV-2 containing solution or an inactivated SARS-CoV-2 containing solution into contact with a cellulose sulfate ester gel at a pH of 8 or more and 10 or less to adsorb the SARS-CoV-2 or the inactivated SARS-CoV-2 to the gel; then removing impurities; and then eluting and recovering the SARS-CoV-2 or the inactivated SARS-CoV-2.

The purification step B is a step of bringing a SARS-CoV-2 containing solution or an inactivated SARS-CoV-2 containing solution into contact with a cellulose sulfate ester gel at a pH of 7 or more and 8 or less to adsorb the SARS-CoV-2 or the inactivated SARS-CoV-2 to the gel; then removing impurities at a pH of 8 or more and 10 or less; and then eluting and recovering the SARS-CoV-2 or the inactivated SARS-CoV-2.

The cellulose sulfate ester gel is a gel formed from cellulose sulfate ester, in which a hydroxyl group of cellulose forms an ester with sulfuric acid. The cellulose sulfate ester is preferably one in which a hydroxyl group at the 6-position of cellulose forms an ester with sulfuric acid. The particle size of the cellulose sulfate ester gel is, for example, 10 µm or more and 200 µm or less, preferably 20 µm or more and 170 µm or less, 30 µm or more and 150 µm or less, and 40 µm or more and 130 µm or less. Such a cellulose sulfate ester gel can be obtained, for example, as Cellufine Sulfate (manufactured by JNC CORPORATION: a product in which the hydroxy group at the 6-position is converted into a sulfate ester, particle size 40 to 130 µm, spherical).

SARS-CoV-2 and an inactivated SARS-CoV-2 can be adsorbed to the cellulose sulfate ester gel at a pH in a range of 7 or more and 10 or less. On the other hand, the production method according to the embodiment is characterized in that SARS-CoV-2 or an inactivated SARS-CoV-2 is adsorbed to the cellulose sulfate ester gel at a pH of 8 or more and 10 or less. When the pH is set in this range, the efficiency of removing impurities derived from the host cells (host cell-derived protein) is improved, exhibiting the advantageous effect that a more highly purified SARS-CoV-2 or inactivated SARS-CoV-2 containing substantially no impurities is obtained. SARS-CoV-2 or an inactivated SARS-CoV-2 is adsorbed to the cellulose sulfate ester gel preferably at a pH of 8.5 or more and 9.5 or less, a pH of 8.6 or more and 9.4 or less, a pH of 8.7 or more and 9.3 or less, a pH of 8.8 or more and 9.2 or less, a pH of 8.9 or more and 9.1 or less, or a pH of 9.

The production method according to another embodiment is characterized in that SARS-CoV-2 or an inactivated SARS-CoV-2 is adsorbed to the cellulose sulfate ester gel at a pH of 7 or more and 8 or less; and impurities are removed from the cellulose sulfate ester gel and the SARS-CoV-2 or the inactivated SARS-CoV-2 are eluted at a pH of 8 or more and 10 or less. When the pH is set in this range, the efficiency of removing impurities derived from the host cells is improved, and further, the yield of SARS-CoV-2 or an inactivated SARS-CoV-2 is also improved, exhibiting the advantageous effect that a highly purified SARS-CoV-2 or inactivated SARS-CoV-2 can be efficiently produced. SARS-CoV-2 or an inactivated SARS-CoV-2 is adsorbed to the cellulose sulfate ester gel preferably at a pH of 7.1 or more and 7.9 or less, a pH of 7.1 or more and 7.8 or less, a pH of 7.2 or more and 7.7 or less, a pH of 7.2 or more and 7.6 or less, a pH of 7.3 or more and 7.5 or less, or a pH of 7.4. Impurities are removed from the cellulose sulfate ester gel and SARS-CoV-2 or an inactivated SARS-CoV-2 is eluted preferably at a pH of 8.5 or more and 9.5 or less, a pH of 8.6 or more and 9.4 or less, a pH of 8.7 or more and 9.3 or less, a pH of 8.8 or more and 9.2 or less, a pH of 8.9 or more and 9.1 or less, or a pH of 9.

Examples of suitable buffers for maintaining the pH in the above range include carbonate bicarbonate buffer (may also be referred to as carbonate buffer), tris-hydrochloride buffer, and glycine-sodium hydroxide buffer. In addition, the concentration of the buffer is usually a concentration used for ion exchange chromatography or the like, for example, 10 mM or more and 100 mM or less, and preferably 10 mM or more and 50 mM or less.

First, a SARS-CoV-2 containing solution or an inactivated SARS-CoV-2 containing solution (for example, concentrated solution) is dialyzed with an appropriate buffer (solvent substitution), and then brought into contact with a cellulose sulfate ester gel that has been equilibrated with the same buffer to adsorb the SARS-CoV-2 or the inactivated SARS-CoV-2. The SARS-CoV-2 containing solution or the inactivated SARS-CoV-2 containing solution may be brought into contact with the cellulose sulfate ester gel by, for example, passing the SARS-CoV-2 containing solution or the inactivated SARS-CoV-2 containing solution through a column packed with the cellulose sulfate ester gel.

The cellulose sulfate ester gel to which the SARS-CoV-2 or the inactivated SARS-CoV-2 has been adsorbed is washed using the buffer used for the adsorption or a similar buffer maintaining the pH.

The SARS-CoV-2 or the inactivated SARS-CoV-2 is eluted from the cellulose sulfate ester gel by increasing the salt concentration of the buffer. The salt concentration is increased by using common salts used for ion exchange, adsorption chromatography, and the like, for example, 0.2M or more and 1M or less of sodium chloride.

The purification step may be performed only once, or may be performed several times. When the purification step is performed several times, the subsequent purification steps after the first time may be performed under the same conditions as in the first purification step, or may be performed under different conditions. When the purification step is performed under different conditions, for example, the pH condition during adsorption is changed between the first purification step and the subsequent purification steps.

### (Formulation Step)

The formulation step is a step of mixing the purified inactivated SARS-CoV-2 with other components to obtain an inactivated SARS-CoV-2 vaccine.

Examples of other components include adjuvants such as aluminum hydroxide, aluminum phosphate, potassium phosphate, mineral oil, and non-mineral oil; and stabilizers such as amino acids and sugars.

The purified inactivated SARS-CoV-2 is replaced with an appropriate buffer by an ultrafiltration method or the like as necessary, and then may be subjected to aseptic filtration with a membrane filter and then mixed with other components; alternatively, may be mixed with other components and then subjected to aseptic filtration with a membrane filter.

Through the production method of the embodiment, an inactivated SARS-CoV-2 vaccine containing substantially no host cell-derived protein is obtained. Here, the phrase "containing substantially no host cell-derived protein" means that the host cell-derived protein is contained in an amount of 10 ng or less, preferably 5 ng or less, 4 ng or less, 3 ng or less, or 2 ng or less, per 1 µg of protein contained in the inactivated SARS-CoV-2 vaccine. The content of protein contained in an inactivated SARS-CoV-2 vaccine can be quantified by performing a protein quantification method (for example, a conventional method such as the TCA-Lowry method) to the vaccine. The content of host cell-derived protein contained in an inactivated SARS-CoV-2 vaccine can be quantified by performing a quantification method capable of specifically detecting the host cell-derived protein (for example, the method described in the examples described below when the host cells are Vero cells) to the vaccine.

Through the production method of another embodiment, a high-purity inactivated SARS-CoV-2 vaccine containing a small amount of host cell-derived protein can be more efficiently produced. Here, the phrase "containing a small amount of host cell-derived protein" means that the host cell-derived protein is contained in an amount of 30 ng or less, preferably 20 ng or less, 18 ng or less, 16 ng or less, 14 ng or less, 12 ng or less, more preferably 10 ng or less, still more preferably 5 ng or less, 4 ng or less, 3 ng or less, or 2 ng or less, per 1 µg of protein contained in the inactivated SARS-CoV-2 vaccine.

### [Purification Method of SARS-CoV-2 or Inactivated SARS-CoV-2]

The purification method of SARS-CoV-2 or an inactivated SARS-CoV-2 of the embodiment includes: a step of bringing a SARS-CoV-2 containing solution or an inactivated SARS-CoV-2 containing solution into contact with a cellulose sulfate ester gel at a pH of 8 to 10 to adsorb the SARS-CoV-2 or the inactivated SARS-CoV-2 to the gel; then removing impurities; and then eluting and recovering the SARS-CoV-2 or the inactivated SARS-CoV-2.

The purification method of SARS-CoV-2 or an inactivated SARS-CoV-2 of another embodiment includes: a step of bringing a SARS-CoV-2 containing solution or an inactivated SARS-CoV-2 containing solution into contact with a cellulose sulfate ester gel at a pH of 7 or more and 8 or less to adsorb the SARS-CoV-2 or the inactivated SARS-CoV-2 to the gel; then removing impurities at a pH of 8 or more and 10 or less; and then eluting and recovering the SARS-CoV-2 or the inactivated SARS-CoV-2.

As a specific embodiment of the purification method according to the embodiment, the embodiments described in the purification step in the production method according to the embodiment can be applied without particular limitation.

Through the purification method of the embodiment, a SARS-CoV-2 antigen composition or an inactivated SARS-CoV-2 antigen composition containing substantially no host cell-derived protein can be obtained. Here, the phrase "containing substantially no host cell-derived protein" means that the host cell-derived protein is contained in an amount of 10 ng or less, preferably 5 ng or less, 4 ng or less, 3 ng or less, or 2 ng or less, per 1 µg of protein contained in the SARS-CoV-2 antigen composition or the inactivated SARS-CoV-2 antigen composition. The content of protein contained in a SARS-CoV-2 antigen composition or an inactivated SARS-CoV-2 antigen composition can be quantified by performing a protein quantification method (for example, a conventional method such as the TCA-Lowry method) to the composition. The content of host cell-derived protein contained in a SARS-CoV-2 antigen composition or an inactivated SARS-CoV-2 antigen composition can be quantified by performing a quantification method capable of specifically detecting the host cell-derived protein (for example, the method described in the examples described below when the host cells are Vero cells) to the composition.

Also through the purification method of another embodiment, a high-purity and high-content SARS-CoV-2 antigen composition or inactivated SARS-CoV-2 antigen composition containing a small amount of host cell-derived protein and containing a large amount of SARS-CoV-2 or an inactivated SARS-CoV-2 can be obtained. Here, the phrase "containing a small amount of host cell-derived protein" means that the host cell-derived protein is contained in an amount of 30 ng or less, preferably 20 ng or less, 18 ng or less, 16 ng or less, 14 ng or less, 12 ng or less, more preferably 10 ng or less, still more preferably 5 ng or less, 4 ng or less, 3 ng or less, or 2 ng or less, per 1 µg of protein contained in the SARS-CoV-2 antigen composition or the inactivated SARS-CoV-2 antigen composition.

Hereinafter, the present invention will be described in detail based on examples, but the present invention is not limited to these examples at all.

### [Example 1: Proliferation of SARS-CoV-2]

Vero cells (CCL-81 (registered trademark)) as a virus proliferation substrate (host cells) were obtained from ATCC (American Type Culture Collection). The Vero cells were proliferated in an appropriate medium and culture method to create a cell bank. The cells in this cell bank were expanded (cell culture) in a D-MEM medium containing bovine serum. Culture was initially carried out in a flask and then expanded into a culture bag. Finally, a Vero cell medium (VP-SFM, manufactured by Thermo Fisher Scientific) containing a cell culture microcarrier (Cytodex (registered trademark), Cytiva (Global Life Sciences Technologies Japan K.K.)) at a concentration of 1 g/L or more and 5 g/L or less was added to a culture tank, and then Vero cells were seeded and proliferated. The proliferated Vero cells were inoculated with a virus (SARS-CoV-2) at an MOI of 0.0001, and the Vero cells infected with the virus were further cultured. From the cultured virus suspension, a virus liquid was harvested.

### [Example 2: Inactivation of SARS-CoV-2]

The harvested virus liquid was concentrated through an ultrafiltration membrane (Hollow fiber cartridge, Cytiva (Global Life Sciences Technologies Japan K.K.)). β-propiolactone was added to the concentrated liquid in a concentration of 0.05 w/v%, and then the liquid was inactivated at 20°C for 16 hours or more to obtain an inactivated SARS-CoV-2 containing solution.

### [Example 3: Purification by Cellulose Sulfate Ester Gel Chromatography (1)]

The inactivated SARS-CoV-2 was purified by column chromatography using a gel packed column (height: 117 mm) in which an empty column (inner diameter 50 mm, Cytiva (Global Life Sciences Technologies Japan K.K.)) is packed with a cellulose sulfate ester gel as a carrier (Cellufine Sulfate, manufactured by JNC CORPORATION: a product in which the hydroxy group at the 6-position is converted into a sulfate ester, particle size 40 to 130 µm, spherical). The column chromatography was performed under the conditions of pH 7.4 and pH 9.0, which are described in Non Patent Literature 2.

Under the condition of pH 7.4, the column was equilibrated with a 10 mM phosphate buffer (pH 7.4) containing 140 mM sodium chloride, and then the solvent of the inactivated SARS-CoV-2 containing solution was replaced with the phosphate buffer (pH 7.4) to obtain an application liquid, and the application liquid (466 mL) was applied to the column so that the inactivated SARS-CoV-2 was adsorbed to the carrier. The phosphate buffer (pH 7.4) was applied to the column, and the carrier after adsorption was washed. The inactivated SARS-CoV-2 was then eluted using a 10 mM phosphate buffer (pH 7.4) containing a higher concentration of sodium chloride (NaCl). The elution was performed stepwise using eluates with stepwise increasing NaCl concentration (0.34 M → 0.6 M → 1 M).

Under the condition of pH 9.0, purification was carried out in the same manner as under the condition of pH 7.4 except that a 10 mM carbonate buffer (pH 9.0) was used in place of a 10 mM phosphate buffer (pH 7.4) containing 140 mM sodium chloride, and the elution was performed stepwise using eluates with stepwise increasing NaCl concentration (0.2 M → 0.4 M → 0.6 M → 0.8 M → 1 M).

The inactivated SARS-CoV-2 content and protein content in the application liquid and the eluate were quantified by the TCA-Lowry method. The host cell-derived protein content in the application liquid and the eluate was quantified by Immunoenzymetric Assay for Measurement of Vero Cell Host Cell Proteins (Catalog#F500, manufactured by CYGNUS Technologies).

The inactivated SARS-CoV-2 yield, protein yield, and host cell-derived protein yield in the eluate were summarized in Table 1. In Table 1, the "yield" is the ratio (%) of the amount contained in the entire eluate to the amount contained in the application liquid.

Under the condition of pH 7.4, the inactivated SARS-CoV-2 yield was 64%, the protein yield was 53%, and the host cell-derived protein yield was 58%. On the other hand, under the condition of pH 9.0, the inactivated SARS-CoV-2 yield was 76%, the protein yield was 64%, and the host cell-derived protein yield was 28%. From this result, when the purification condition at pH 9.0 is used, the host cell-derived protein yield is significantly reduced with respect to the inactivated SARS-CoV-2 yield and the protein yield. That is, it was confirmed that in the purification condition at pH 9.0, an eluate from which the host cell-derived protein in the application liquid was further removed was obtained, and the amount of impurities was small and the purity was high.

**[Table 1]**

| | Inactivated SARS-CoV-2 yield | Protein yield | Host cell-derived protein yield |
|---|---|---|---|
| pH 7.4 | 64% | 53% | 58% |
| pH 9.0 | 76% | 64% | 28% |

### [Example 4: Purification by Cellulose Sulfate Ester Gel Chromatography (2)]

The purification by ion exchange chromatography usually used in the production of an inactivated SARS-CoV-2 vaccine was compared with the purification by chromatography using a cellulose sulfate ester gel as a carrier.

### (Purification by Cellulose Sulfate Ester Gel Chromatography)

A column (inner diameter 140 mm, length 150 mm, Cytiva (Global Life Sciences Technologies Japan K.K.)) packed with a cellulose sulfate ester gel as a carrier (Cellufine Sulfate, manufactured by JNC CORPORATION: a product in which the hydroxy group at the 6-position is converted into a sulfate ester, particle size 40 to 130 µm, spherical) was repeatedly washed with a 10 mM carbonate buffer adjusted to pH 9.0 to be equilibrated. Then, the inactivated SARS-CoV-2 containing solution replaced with the buffer was applied to the column, and the inactivated SARS-CoV-2 was adsorbed to the carrier. The inactivated SARS-CoV-2 was eluted by slowly adding a 1 M sodium chloride solution. The recovered eluate was replaced with a 10 mM phosphate buffer (pH 7.2) containing 140 mM sodium chloride by an ultrafiltration method or the like to prepare an original drug for vaccine production (inactivated SARS-CoV-2 antigen composition).

### (Purification by Ion Exchange Chromatography)

As a carrier for ion exchange chromatography, an anion chromatography membrane (Sartobind Q, manufactured by Sartorius AG) was used. An inactivated SARS-CoV-2 containing solution that has been adjusted to have a sodium chloride concentration of 340 mM was applied to the anion chromatography membrane equilibrated with a 340 mM sodium chloride phosphate buffer, and the flow-through solution was recovered. The recovered flow-through solution was replaced with a 10 mM phosphate buffer (pH 7.2) containing 140 mM sodium chloride by an ultrafiltration method or the like to prepare an original drug for vaccine production (inactivated SARS-CoV-2 antigen composition).

The protein content and the host cell-derived protein content in each original drug were quantified in an ELISA system using an anti-Vero protein antibody produced by immunizing rabbits and guinea pigs with a protein obtained from the Vero cell culture supernatant. The results are shown in Table 2. In Table 2, the host cell-derived protein content per 1 dose indicates the host cell-derived protein content when 1 dose is defined as a protein content of 10 µg.

**[Table 2]**

| | Cellulose sulfate ester gel chromatography | Ion exchange chromatography |
|---|---|---|
| Protein content (µg/mL) | 129.2 | 148.3 |
| Host cell-derived protein content (ng/mL) | 189.8 | 5002.6 |
| Host cell-derived protein content per 1 dose (ng) | 14.7 | 337.3 |

The host cell-derived protein content in the original drug obtained using an anion chromatography membrane as a carrier was 337.3 ng per 1 dose, whereas the host cell-derived protein content in the original drug obtained using a cellulose sulfate ester gel was 14.7 ng per 1 dose. That is, it was shown that the original drug (inactivated SARS-CoV-2 antigen composition) obtained by purification through chromatography using a cellulose sulfate ester gel as a carrier had less impurities and high purity.

### [Example 5: Production and Evaluation of Inactivated SARS-CoV-2 Vaccine]

The original drug (inactivated SARS-CoV-2 antigen composition) obtained by purification through chromatography using a cellulose sulfate ester gel as a carrier in Example 4 was directly used as an inactivated SARS-CoV-2 vaccine (non-adjuvanted vaccine). To the original drug, aluminum hydroxide was added as an adjuvant to obtain an inactivated SARS-CoV-2 vaccine (adjuvanted vaccine). Balb/c mice (N = 6) and C57BL/6N mice (N = 6) were intramuscularly administered with the adjuvanted vaccine or the non-adjuvanted vaccine twice at an interval of 2 weeks, at a dose of 0.0625 µg/dose, 0.25 µg/dose, or 1.00 µg/dose, and 14 days after the second administration, the neutralizing antibody value against SARS-CoV-2 in the serum was measured. The measurement results are shown in FIG. 1. As shown in FIG. 1, it was found that when a mouse was immunized with the vaccine, a neutralizing antibody with high titer against SARS-CoV-2 was induced in the serum of the mouse.

### [Example 6: Purification by Cellulose Sulfate Ester Gel Chromatography (3)]

The column packed with a cellulose sulfate ester gel used in Example 4 was repeatedly washed with a 10 mM carbonate buffer adjusted to pH 9.0 to be equilibrated. Then, the inactivated SARS-CoV-2 containing solution replaced with a 50 mM phosphate buffer (pH 7.4) containing 100 mM sodium chloride was applied to the column, and the inactivated SARS-CoV-2 was adsorbed to the carrier. Thereafter, five column volume washing was performed with a 10 mM carbonate buffer adjusted to pH 9.0 for equilibration. The inactivated SARS-CoV-2 was eluted by slowly adding a 1 M sodium chloride solution (pH 9.0). The recovered eluate was replaced with a 10 mM phosphate buffer (pH 7.2) containing 140 mM sodium chloride by an ultrafiltration method or the like to prepare an original drug for vaccine production (inactivated SARS-CoV-2 antigen composition).

The protein content and the host cell-derived protein content in the original drug were quantified in an ELISA system using an anti-Vero protein antibody produced by immunizing rabbits and guinea pigs with a protein obtained from the Vero cell culture supernatant. The results are shown in Table 3 in comparison with the results of Example 4. In Table 3, the host cell-derived protein content per 1 dose indicates the host cell-derived protein content when 1 dose is defined as a protein content of 10 µg.

At the time of adsorption, the pH of the inactivated SARS-CoV-2 containing solution was set to 7.4, so that the host cell-derived protein content was 152.4 ng per 1 dose. As compared with the result of Example 4 (14.7 ng), in which the pH of the inactivated SARS-CoV-2 containing solution was 9.0, a large amount of impurity was contained during adsorption, but the impurities were reduced as compared with the result when an anion chromatography membrane was used as a carrier (337.3 ng). On the other hand, focusing on the protein content, when the pH of the inactivated SARS-CoV-2 containing solution was 7.4 during adsorption, the result was 408.1 µg/mL, which was larger than the result of Example 4 (129.2 µg/mL), and it was found that the method of this example is effective in order to increase the protein yield.

**[Table 3]**

| | Cellulose sulfate ester gel chromatography | | Ion exchange chromatography |
|---|---|---|---|
| | pH 7.4 during adsorption (Example 6) | pH 9.0 during adsorption (Example 4) | |
| Protein content (µg/mL) | 408.1 | 129.2 | 148.3 |
| Host cell-derived protein content (ng/mL) | 6220 | 189.8 | 5002.6 |
| Host cell-derived protein content per 1 dose (ng) | 152.4 | 14.7 | 337.3 |

## Claims

1. A production method of an inactivated SARS-CoV-2 vaccine, the production method comprising:
a step of bringing a SARS-CoV-2 comprising solution or an inactivated SARS-CoV-2 comprising solution into contact with a cellulose sulfate ester gel at a pH of 8 or more and 10 or less to adsorb the SARS-CoV-2 or the inactivated SARS-CoV-2 to the gel; then removing impurities; and then eluting and recovering the SARS-CoV-2 or the inactivated SARS-CoV-2.

2. An inactivated SARS-CoV-2 vaccine obtained by the production method according to claim 1, and comprising substantially no host cell-derived protein.

3. The vaccine according to claim 2, further comprising an adjuvant.

4. A purification method of SARS-CoV-2 or an inactivated SARS-CoV-2, the purification method comprising:
a step of bringing a SARS-CoV-2 comprising solution or an inactivated SARS-CoV-2 comprising solution into contact with a cellulose sulfate ester gel at a pH of 8 or more and 10 or less to adsorb the SARS-CoV-2 or the inactivated SARS-CoV-2 to the gel; then removing impurities; and then eluting and recovering the SARS-CoV-2 or the inactivated SARS-CoV-2.

5. A SARS-CoV-2 antigen composition or an inactivated SARS-CoV-2 antigen composition obtained by the purification method according to claim 4, and comprising substantially no host cell-derived protein.

6. A production method of an inactivated SARS-CoV-2 vaccine, the production method comprising:
a step of bringing a SARS-CoV-2 comprising solution or an inactivated SARS-CoV-2 comprising solution into contact with a cellulose sulfate ester gel at a pH of 7 or more and 8 or less to adsorb the SARS-CoV-2 or the inactivated SARS-CoV-2 to the gel; then removing impurities at a pH of 8 or more and 10 or less; and then eluting and recovering the SARS-CoV-2 or the inactivated SARS-CoV-2.

7. An inactivated SARS-CoV-2 vaccine obtained by the production method according to claim 6.

8. The vaccine according to claim 7, wherein a content of host cell-derived protein is 30 ng or less with respect to 1 µg of protein comprised in the inactivated SARS-CoV-2 vaccine.

9. The vaccine according to claim 7 or 8, further comprising an adjuvant.

10. A purification method of SARS-CoV-2 or an inactivated SARS-CoV-2, the purification method comprising:
a step of bringing a SARS-CoV-2 comprising solution or an inactivated SARS-CoV-2 comprising solution into contact with a cellulose sulfate ester gel at a pH of 7 or more and 8 or less to adsorb the SARS-CoV-2 or the inactivated SARS-CoV-2 to the gel; then removing impurities at a pH of 8 or more and 10 or less; and then eluting and recovering the SARS-CoV-2 or the inactivated SARS-CoV-2.

11. A SARS-CoV-2 antigen composition or an inactivated SARS-CoV-2 antigen composition obtained by the purification method according to claim 10.

12. The antigen composition according to claim 11, wherein a content of host cell-derived protein is 30 ng or less with respect to 1 µg of protein comprised in the SARS-CoV-2 antigen composition or the inactivated SARS-CoV-2 antigen composition.
